# FASCICULE DE BREVET EUROPEEN

(11) **EP 1 605 994 B1**
(45) Date de publication et mention de la délivrance du brevet: **31.10.2018**
(21) Numéro de dépôt: 04742276.1
(22) Date de dépôt: 18.03.2004
(51) Int. Cl.: A61M 5/20, A61M 5/31, A61M 5/48, A61M 5/30

(54) **DISPOSITIF D'INJECTION SANS AIGUILLE A CARTOUCHE PYROTECHNIQUE**
NADELLOSE INJEKTIONSVORRICHTUNG MIT EINER PYROTECHNISCHEN KARTUSCHE
NEEDLELESS INJECTION DEVICE COMPRISING A PYROTECHNIC CARTRIDGE AND METHOD OF ASSEMBLING ONE SUCH DEVICE

(30) Priorité: 21.03.2003 FR 0303497
(43) Date de publication de la demande: 21.12.2005
(73) Titulaire: Crossject, 21000 Dijon (FR)
(72) Inventeur: ALEXANDRE, Patrick, F-70100 Gray (FR); BAUD, Georges, F-83260 La Crau (FR); BROUQUIERES, Bernard, F-83100 Toulon (FR); GAUTIER, Philippe, F-91220 Le Plessis Pate (FR)
(74) Mandataire: Gaillarde, Frédéric F. Ch.
(86) Numéro de dépôt international: PCT/FR2004/000658
(87) Numéro de publication internationale: WO 2004/084975

(56) Documents cités:
- WO-A-97/47730
- FR-A- 2 815 544
- US-A- 5 520 639
- US-B1- 6 328 714

## Description

Le domaine technique de l'invention est celui des dispositifs d'injection sans aiguille préremplis et jetables, fonctionnant avec un générateur de gaz, et utilisés pour les injections intradermiques, sous-cutanées et intramusculaires, de principe actif liquide à usage thérapeutique en médecine humaine ou vétérinaire.

Le principe actif est constitué par un liquide plus ou moins visqueux, un mélange de liquide, ou un gel. Le principe actif peut également être un solide mis en solution dans un solvant approprié pour l'injection ou être constitué d'un solide pulvérulent mis en suspension à une certaine concentration dans un liquide approprié. La granulométrie du principe actif doit alors être compatible avec le diamètre des conduits pour éviter de les obturer.

Dans l'art antérieur, des dispositifs d'injection sans aiguille ont déjà fait l'objet de plusieurs dépôts de demandes de brevets.

La demande de brevet WO 00/48654 est relative à un dispositif d'injection sans aiguille jetable permettant d'injecter une quantité modulable de principe actif liquide. Ce dispositif comporte plus particulièrement un réservoir de liquide dans lequel est placé un piston apte à pousser le liquide à travers un système d'injection. Ce dispositif comporte une réserve de gaz et un dispositif permettant de percer cette réserve de gaz de manière à libérer les gaz nécessaires pour pousser le piston présent dans le réservoir de liquide et ainsi à éjecter le liquide hors du dispositif. Suivant la nature et/ou la quantité de principe actif liquide à injecter en fonction du traitement ainsi que suivant la profondeur de pénétration à travers la peau, souhaitée pour ledit principe actif, il est nécessaire de pouvoir adapter la quantité de gaz à générer. Or, lorsqu'il s'agit d'un dispositif d'injection sans aiguille prérempli prêt à l'emploi pour l'utilisateur, ce choix devra être effectué définitivement lors du processus d'assemblage du dispositif.

Dans le processus d'assemblage d'un dispositif tel que celui divulgué dans la demande de brevet WO 00/48654, la réserve de gaz est positionnée, dans une première étape, dans le corps du dispositif puis ensuite, dans une étape ultérieure, le réservoir de liquide à injecter est fixé sur ledit corps. L'ordre obligatoire de ces deux étapes est particulièrement contraignant dans la mesure où il est impossible d'adapter facilement la quantité de gaz présente dans la réserve de gaz à la nature et/ou à la quantité de liquide à injecter ainsi qu'à la profondeur de pénétration souhaitée.

Le brevet US 4,941,880 divulgue un dispositif d'injection sans aiguille dans lequel une réserve de gaz vient se visser à une extrémité du dispositif indépendamment du réservoir de liquide. Toutefois, l'assemblage de la réserve de gaz sur le dispositif ne peut être effectué que lorsque le système de perçage de la réserve de gaz est disposé dans le corps. Cette contrainte devra donc être prise en compte lors de l'assemblage d'un tel dispositif.

De plus, dans un tel dispositif, il peut s'avérer dangereux de fixer la réserve de gaz à une extrémité de ce dispositif, celle-ci étant alors facilement accessible à l'utilisateur et susceptible de se détériorer.

En outre, il est connu des documents US 5,520,639 et US 6,328,714, de fournir un dispositif d'injection sans aiguille comportant une cartouche de gaz compressée. De tels dispositifs ne sont pas entièrement jetables et ne sont pas ailleurs pas adaptés pour recevoir des cartouches pyrotechniques.

Enfin, le document FR 2 815 544 divulgue un dispositif d'injection sans aiguille comportant notamment une charge pyrotechnique et un percuteur. Ce dispositif est susceptible d'améliorations, notamment dans le but d'augmenter la longueur de course du percuteur, sans augmenter les dimensions extérieures du dispositif.

Un but de l'invention est de ne disposer, lors du processus d'assemblage d'un dispositif d'injection sans aiguille, d'aucune contrainte liée à l'assemblage de la partie génératrice de gaz sur le dispositif. Un autre but de l'invention est d'éviter que l'assemblage de la partie génératrice de gaz sur le dispositif ne soit réalisé de manière à rendre la partie génératrice de gaz facilement accessible à l'utilisateur sur le dispositif une fois entièrement assemblé.

Ce but est atteint par un dispositif d'injection sans aiguille, selon la revendication 1.

Selon l'invention, il sera donc possible, en jouant sur la nature et/ou la quantité de gaz à générer, de réaliser sur une même chaîne de montage des dispositifs d'injections sans aiguille dans lesquels diffèrent la nature et/ou la quantité de principe actif liquide à injecter. La quantité de gaz à générer doit également être adaptée en fonction de la profondeur de pénétration à obtenir pour le principe actif à injecter.

Selon l'invention, il n'existe donc aucune contrainte liée à la nature et/ ou à la quantité du principe actif liquide destinée à être placé dans le dispositif, ainsi qu'à la profondeur de pénétration à travers la peau, souhaitée pour ledit principe actif. Ainsi, il sera possible de personnaliser le dispositif d'injection sans aiguille au plus tard lors de son processus d'assemblage, c'est-à-dire d'adapter facilement la quantité de gaz à générer en fonction de la nature et/ ou de la quantité de principe actif à injecter et de la profondeur de pénétration souhaitée pour ledit principe actif. De plus, la cartouche génératrice de gaz est insérée dans le circuit d'éléments, dans un logement du corps et n'est donc pas facilement accessible à l'utilisateur.

Selon un mode de réalisation préféré, le corps comporte une ouverture communiquant avec le logement. Selon l'invention, le corps comporte donc une ouverture spécifique indépendante communiquant avec le logement dans laquelle est insérée la cartouche génératrice de gaz. Ainsi, la cartouche génératrice de gaz pourra être positionnée dans le circuit d'éléments à n'importe quelle étape du processus d'assemblage du corps du dispositif et ceci indépendamment de l'assemblage des autres éléments sur le corps.

Selon une autre particularité, le logement est placé entre le dispositif d'initiation et le réservoir contenant le principe actif liquide.

Selon un mode de réalisation préféré, la cartouche a la forme d'un conduit contribuant, une fois en place dans le logement, à former une liaison entre les éléments situés en amont et les éléments situés en aval.

Selon une autre particularité, le circuit d'éléments suit une forme en U renversé comportant donc deux branches parallèles reliées entre elle par une branche transversale perpendiculaire.

Selon une autre particularité, l'introduction de la cartouche dans le circuit est réalisée perpendiculairement à l'axe de symétrie du U formé par le circuit.

Selon une autre particularité, la cartouche a une forme en L et en ce que, une fois insérée, sa forme suit un angle droit présent entre l'une des branches parallèles du U renversé formé par le circuit et sa branche transversale.

Selon un mode de réalisation préféré, la cartouche génératrice de gaz est une cartouche pyrotechnique comprenant une charge pyrotechnique. Selon l'invention, l'insertion de la charge pyrotechnique dans le dispositif pourra se faire à n'importe quelle étape du processus d'assemblage du corps du dispositif et notamment vers la fin de ce processus ce qui permettra à la fois d'adapter la charge pyrotechnique à la profondeur de pénétration souhaitée ainsi qu'à la nature et/ou à la quantité de principe actif présent dans le dispositif, mais également de limiter les manipulations de la charge pyrotechnique au cours du processus d'assemblage du dispositif et ainsi de réduire les risques d'initiation intempestive des charges tout au long du processus d'assemblage.

Selon une particularité de ce dernier mode de réalisation préféré, la cartouche comporte une amorce.

Selon une autre particularité, la cartouche a la forme d'un conduit en L dans lequel est placée la charge pyrotechnique, ce conduit étant obturé à l'une de ses extrémités par l'amorce et à son autre extrémité par un opercule frangible.

Selon une autre particularité, le dispositif d'initiation de la charge pyrotechnique comporte un dispositif de percussion de l'amorce. Le dispositif de percussion sera par exemple constitué d'un percuteur actionné à l'aide d'un ressort.

Selon une autre particularité, le logement du corps, apte à recevoir la cartouche, est placé entre le dispositif de percussion et une chambre d'expansion des gaz située en amont du réservoir.

Selon une autre particularité, le corps comprend une première partie creuse et une deuxième partie creuse disposées suivant deux axes parallèles et reliées par un conduit, ce conduit délimitant le logement de la cartouche et la chambre d'expansion des gaz.

Selon une autre particularité, la cartouche est placée dans le logement du corps de sorte que l'amorce soit située dans l'axe du dispositif de percussion et que l'opercule soit situé dans l'axe de la chambre d'expansion des gaz.

L'invention, avec ses caractéristiques et avantages, ressortira plus clairement à la lecture de la description faite en référence aux dessins annexés dans lesquels :
La figure 1 représente en perspective et en mode éclaté le corps du dispositif ainsi que certains éléments destinés à être assemblés sur le corps du dispositif.
La figure 2 représente en perspective le corps du dispositif sur lequel certains éléments ont été assemblés ainsi que la cartouche génératrice de gaz.
La figure 3 représente en perspective et en mode éclaté le réservoir destiné à recevoir le principe actif liquide.
La figure 4 représente en perspective et en mode éclaté une cartouche pyrotechnique génératrice de gaz utilisée dans le dispositif selon l'invention.
La figure 5 représente en perspective un opercule frangible tel qu'utilisé dans la cartouche pyrotechnique de la figure 4.
La figure 6 représente en coupe longitudinale partielle un dispositif d'injection sans aiguille selon l'invention en position non activé, dans lequel est inséré la cartouche pyrotechnique représentée en figure 4.
La figure 7 représente en coupe longitudinale le corps du dispositif ayant fonctionné et dans lequel est visible une cartouche pyrotechnique vide 4.

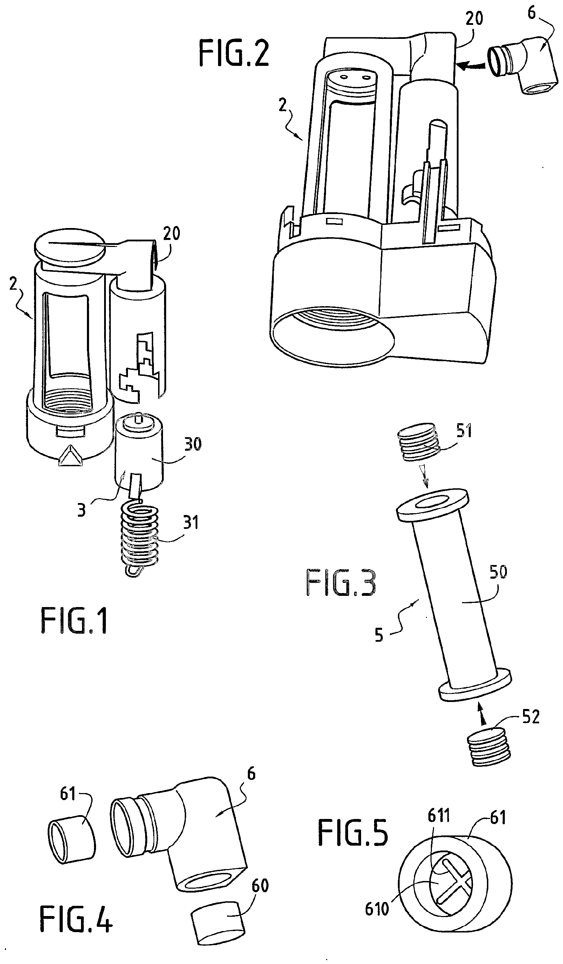

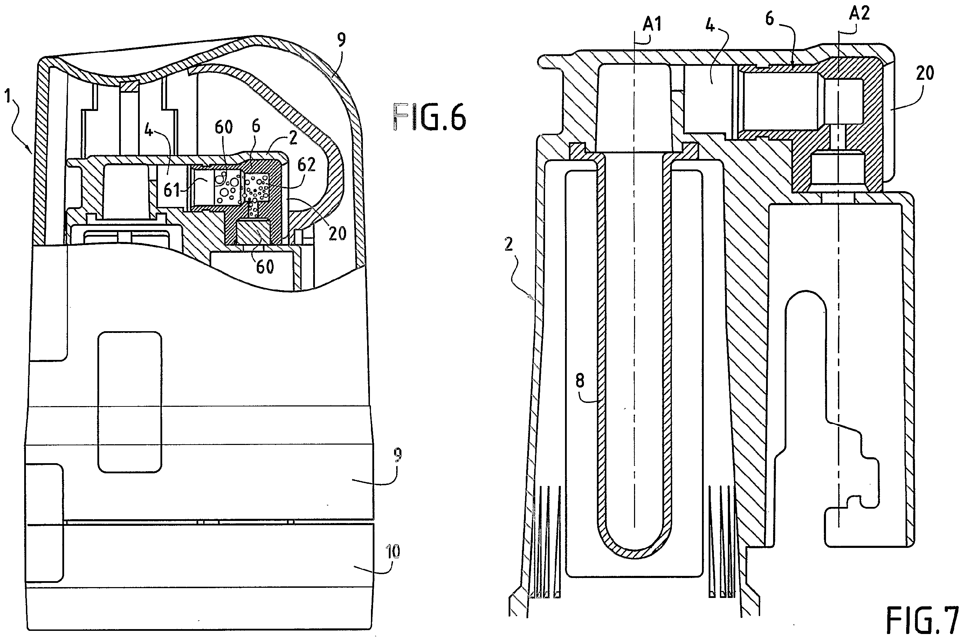

Un dispositif 1 d'injection sans aiguille selon l'invention, représenté en figure 6, comporte un corps 2 creux en forme de U renversé inséré sous un capot 9 d'actionnement du dispositif 1, ce capot étant obturé par un bouchon 10. Cette forme en U confère au dispositif une forme compacte dont les avantages sont plus particulièrement décrits dans le brevet n° FR 2 815 544. L'actionnement d'un tel dispositif 1 par le patient à l'aide du capot 9 est également décrit dans le brevet FR 2 815 544. Lors du processus d'assemblage du dispositif 1, ce corps 2 est destiné à recevoir une pluralité d'éléments. Ainsi, une fois assemblé, le corps 2, représenté en figure 1, comporte ou délimite successivement, de l'amont vers l'aval, un dispositif 3 de percussion comprenant un percuteur 30 et un ressort 31, une amorce 60, une charge 62 pyrotechnique, ces trois éléments formant un générateur de gaz, une chambre 4 d'expansion des gaz, un réservoir 5 (figure 3) contenant un principe actif liquide à injecter et un système d'injection (non visible). Le générateur de gaz constitue un premier sous-ensemble linéaire inséré dans le corps 2 suivant une première branche verticale du U renversé formé par le corps 2. Le réservoir 5 contenant le principe actif à injecter et le système d'injection forment un second sous-ensemble linéaire inséré suivant la seconde branche verticale du U renversé formé par le corps 2. Le premier et le second sous-ensembles sont linéaires suivant deux axes (A1, A2, figure 7) parallèles et sont reliés entre eux par la chambre 4 d'expansion des gaz qui est formée dans le corps 2 suivant un axe perpendiculaire aux axes (A1, A2) des deux sous-ensembles, c'est-à-dire suivant la branche transversale reliant les deux branches parallèles du U renversé formé par le corps 2.

Le réservoir 5 représenté en figure 3 est par exemple constitué d'un tube 50 en verre ouvert à ses deux extrémités. Le tube 50 est inséré dans le corps 2 de manière à être relié à son extrémité la plus en amont à la chambre 4 d'expansion des gaz et à son extrémité la plus en aval au système d'injection. Le principe actif (non représenté) est par exemple emprisonné dans le tube 50 en verre entre un bouchon-piston amont 51 et un bouchon-piston aval 52 insérés dans le tube 50. Les bouchons-pistons amont 51 et aval 52 sont réalisés par exemple dans un matériau déformable à base d'élastomère. Le système d'injection comporte notamment une buse d'injection à travers laquelle est injecté le principe actif contenu dans le réservoir 5. Cette buse d'injection comprend par exemple une pluralité de canaux d'injection destinés à être traversés par le liquide lors de l'injection.

Selon l'invention, le générateur de gaz comporte une cartouche 6 génératrice de gaz et un dispositif 3 de percussion. La cartouche 6 génératrice de gaz représentée en figures 2 et 4 est par exemple métallique et comporte une amorce 60 et une charge 62 (Figure 6) pyrotechnique permettant de générer la quantité de gaz nécessaire pour provoquer l'injection du principe actif. L'amorce 60 est par exemple du type de celle utilisée dans une cartouche pour fusil de chasse. La charge 62 pyrotechnique est constituée d'une poudre apte à émettre une grande quantité de gaz comme, par exemple, une poudre simple base à la nitrocellulose. En référence à la figure 4, la cartouche 6 génératrice de gaz utilisée dans le dispositif 1 d'injection sans aiguille selon l'invention se présente par exemple sous la forme d'un conduit en forme de L dans lequel est placée la charge 62 pyrotechnique. Lorsque la cartouche 6 est encastrée dans le dispositif 1 comme représenté en figure 6, l'extrémité la plus en amont du conduit formant la cartouche est obturée par l'amorce 60 tandis que l'extrémité la plus en aval de ce conduit est obturée par un opercule 61 frangible représenté plus en détail en figure 5. Cet opercule 61 se présente sous la forme d'un bouchon cylindrique enfoncé dans le canal du conduit formé par la cartouche 6. Ce bouchon comporte une paroi 610, perpendiculaire à l'axe du conduit et obturant le conduit, sur laquelle est formée une amorce 611 de rupture. L'amorce 611 de rupture constitue une zone de fragilisation suivant laquelle, sous une certaine pression des gaz, l'opercule 61 cède et s'ouvre en formant des pétales. Le seuil de claquage ou d'ouverture de l'opercule frangible est déterminé par la profondeur de l'amorce 611 de rupture formée sur la paroi 610. La charge 62 pyrotechnique est placée dans le conduit formé par la cartouche 6 entre l'amorce 60 et l'opercule 61 frangible. Sur la figure 7, est représenté le corps 2 d'un dispositif ayant fonctionné et dans lequel la cartouche 6 est vide.

Selon l'invention, le corps 2 comporte, entre le dispositif 3 de percussion et la chambre 4 d'expansion, un logement accessible de l'extérieur du corps 2 et destiné à recevoir la cartouche 6 génératrice de gaz. Ce logement suit l'angle droit défini entre la chambre 4 d'expansion des gaz et la première branche verticale du U formé par le corps 2. Une ouverture 20 communiquant avec le logement est formée sur le corps 2. Cette ouverture 20 est formée latéralement sur le corps 2, sensiblement dans l'axe de la chambre 4 d'expansion des gaz. La cartouche 6 génératrice de gaz est destinée à être insérée dans ladite ouverture 20 jusqu'à venir s'encastrer dans le logement prévu pour elle. La cartouche 6 génératrice de gaz est insérée de sorte que sa forme en L suive l'angle droit formé entre la première branche verticale du U renversé formé par le corps 2 et la chambre 4 d'expansion des gaz. La cartouche 6 une fois encastrée dans le logement est sertie sur le corps 2 au niveau de l'ouverture 20. Une fois en place dans le logement, l'opercule 61 frangible obturant le conduit formé par la cartouche 6 à son extrémité aval se trouve dans l'axe de la chambre 4 d'expansion des gaz et l'amorce 60 obturant ledit conduit à son extrémité amont se trouve dans l'axe du premier sous-ensemble et plus particulièrement dans l'axe du percuteur 30.

En rendant accessible de l'extérieur du corps 2 le logement de la cartouche 6 pyrotechnique, cela permet, lors du processus d'assemblage du dispositif, de positionner la cartouche 6 dans le corps 2 à n'importe quel stade de ce processus. Selon l'invention, le positionnement de la cartouche 6 dans le corps 2 est effectué indépendamment de l'assemblage des autres éléments du dispositif, c'est-à-dire, par exemple, qu'il n'est pas nécessaire que la cartouche 6 soit placé dans le dispositif antérieurement au dispositif 3 de percussion.

Ainsi, selon l'invention, il sera donc possible d'adapter la cartouche 6 pyrotechnique en fonction de la nature et/ou de la quantité de principe actif liquide à injecter ainsi qu'en fonction de la profondeur de pénétration à travers la peau, souhaitée pour ledit principe actif. De plus, il pourra s'avérer intéressant de pouvoir placer la cartouche 6 dans le dispositif vers la fin du processus d'assemblage pour éviter les manipulations de la cartouche 6, ces manipulations pouvant causer l'initiation intempestive de la charge 62 pyrotechnique.

Le fonctionnement d'un tel dispositif 1 d'injection sans aiguille ayant des composants tels que ceux définis dans la présente demande est décrit en détail dans la demande de brevet français FR 2 815 544. Le fonctionnement global d'un tel dispositif 1 peut toutefois être résumé de la manière suivante :
Au repos, le percuteur 30 est par exemple en appui contre une butée à l'aide du ressort 31 précontraint dont l'axe est sensiblement confondu avec l'axe du percuteur 30. Une manipulation du patient provoque la libération du percuteur 30 qui, sous l'effet de la détente du ressort, vient percuter l'amorce 60 située dans le même axe. L'initiation de l'amorce 60 entraîne ensuite l'allumage de la charge 62 pyrotechnique contenue dans la cartouche 6. Lorsqu'une certaine pression de gaz est atteinte, l'opercule 61 frangible s'ouvre suivant son amorce 611 de rupture et laisse ainsi passer les gaz dans la chambre 4 d'expansion. Les gaz permettent ensuite, en poussant sur le bouchon-piston amont 51 contenu dans le tube 50, d'éjecter le principe actif liquide à travers le système d'injection. Comme représenté sur la figure 7, pour éviter que les gaz ne viennent directement au contact du bouchon-piston amont 51 et ainsi éviter de polluer le principe actif liquide contenu dans le tube 50, une membrane 8 souple peut être prévue à la sortie de la chambre 4 d'expansion des gaz. Cette membrane 8 souple, sous l'action des gaz, peut se déployer à l'intérieur du tube 50 pour venir pousser le bouchon-piston amont 51 présent dans le tube 50 et ainsi provoquer l'éjection du principe actif liquide à travers le système d'injection. Cette membrane souple constitue une paroi étanche entre les gaz générés et le principe actif. Sur la figure 7, le corps 2 représenté est celui d'un dispositif ayant déjà fonctionné, c'est-à-dire dans lequel la membrane 8 a été déployée et la cartouche 6 a été vidée après la combustion de la totalité de la charge 62 pyrotechnique.

Il doit être évident pour les personnes versées dans l'art que la présente invention permet des modes de réalisation sous de nombreuses autres formes spécifiques sans l'éloigner du domaine d'application de l'invention comme revendiqué. Par conséquent, les présents modes de réalisation doivent être considérés à titre d'illustration, mais peuvent être modifiés dans le domaine défini par la portée des revendications jointes, et l'invention ne doit pas être limitée aux détails donnés ci-dessus.

## Revendications

1. Dispositif (1) d'injection sans aiguille comprenant un corps (2) supportant ou délimitant une pluralité d'éléments formant un circuit d'éléments, ce circuit comprenant un dispositif d'initiation, une cartouche (6) génératrice de gaz, un réservoir (5) contenant un principe actif à injecter et un système d'injection du principe actif, le corps (2) comportant un logement destiné à recevoir ladite cartouche (6), ledit logement étant accessible de l'extérieur de manière à pouvoir insérer directement la cartouche (6) dans le circuit d'éléments, indépendamment des autres éléments, la cartouche (6) génératrice de gaz étant une cartouche (6) pyrotechnique comprenant une charge (62) pyrotechnique, ladite cartouche (6) comportant une amorce (60),
le corps (2) comportant une ouverture communiquant avec le logement,
le circuit d'éléments suivant une forme en U renversé comportant deux branches parallèles reliées entre elles par une branche transversale perpendiculaire,
l'introduction de la cartouche (6) dans le circuit étant réalisée perpendiculairement à l'axe de symétrie du U formé par le circuit,
ce dispositif étant **caractérisé en ce que** la cartouche (6) a une forme de conduit en L et **en ce que**, une fois insérée, sa forme suit un angle droit présent entre l'une des branches parallèles du U renversé formé par le circuit et sa branche transversale, et **en ce que** ledit conduit est obturé à l'une de ses extrémités par l'amorce (60) et à son autre extrémité par un opercule (61) frangible.

2. Dispositif (1) selon la revendication 1, **caractérisé en ce que** la cartouche (6), une fois en place dans le logement, obture l'ouverture (20) de manière étanche par rapport à l'extérieur.

3. Dispositif (1) selon l'une des revendications 1 ou 2, **caractérisé en ce que** le logement est placé entre le dispositif d'initiation et le réservoir (5) contenant le principe actif liquide.

4. Dispositif (1) selon l'une quelconque des revendications précédentes, **caractérisé en ce que** le dispositif d'initiation de la charge (62) pyrotechnique comporte un dispositif (3) de percussion de l'amorce.

5. Dispositif (1) selon la revendication 4, **caractérisé en ce que** le logement du corps (2), apte à recevoir la cartouche (6), est placé entre le dispositif (3) de percussion et une chambre (4) d'expansion des gaz située en amont du réservoir (5).

6. Dispositif (1) selon la revendication 5, **caractérisé en ce que** le corps (2) comprend une première partie creuse et une deuxième partie creuse disposées suivant deux axes parallèles (A1, A2) et reliées par un conduit, ce conduit délimitant le logement de la cartouche (6) et la chambre (4) d'expansion des gaz.

7. Dispositif (1) selon la revendication 6, **caractérisé en ce que** la cartouche (6) est placée dans le logement du corps (2) de sorte que l'amorce (60) soit située dans l'axe du dispositif (3) de percussion et que l'opercule (61) soit situé dans l'axe de la chambre (4) d'expansion des gaz.

## Patentansprüche

1. Nadellose Injektionsvorrichtung (1), umfassend einen Körper (2), der eine Vielzahl von Elementen trägt oder abgrenzt, die einen Elementekreis bilden, wobei dieser Kreis eine Zündvorrichtung, eine Gas erzeugende Patrone (6), ein Reservoir (5), das einen zu injizierenden Wirkstoff enthält, und ein System zum Injizieren des Wirkstoffs umfasst, wobei der Körper (2) eine Aufnahme umfasst, die dazu vorgesehen ist, die Patrone (6) aufzunehmen, wobei die Aufnahme von der Außenseite her zugänglich ist, um die Patrone (6) unabhängig von den anderen Elementen direkt in den Elementekreis einsetzen zu können, wobei die Gas erzeugende Patrone (6) eine pyrotechnische Patrone (6) ist, die eine pyrotechnische Ladung (62) umfasst, wobei die Patrone (6) einen Zünder (60) umfasst,
wobei der Körper (2) eine Öffnung umfasst, die mit der Aufnahme kommuniziert,
wobei der Elementekreis einer umgekippten U-Form folgt, die zwei über einen senkrechten Querschenkel miteinander verbundene parallele Schenkel umfasst,
wobei das Einführen der Patrone (6) in den Kreis senkrecht zur Symmetrieachse des vom Kreis gebildeten U ausgeführt wird,
wobei diese Vorrichtung **dadurch gekennzeichnet ist, dass** die Patrone (6) eine L-förmige Leitungsform aufweist und dadurch, dass sobald sie eingesetzt ist, ihre Form einem rechten Winkel folgt, der zwischen einem der parallelen Schenkel des umgekippten U, das vom Kreis gebildet wird, und dessen Querschenkel vorhanden ist, und dadurch, dass die Leitung an einem ihrer Enden vom Zünder (60) und an ihrem anderen Ende von einer brechbaren Kappe (61) verschlossen wird.

2. Vorrichtung (1) nach Anspruch 1, **dadurch gekennzeichnet, dass** die Patrone (6), sobald sie sich in der Aufnahme an Ort und Stelle befindet, die Öffnung (20) in Bezug auf die Außenseite in dichter Weise verschließt.

3. Vorrichtung (1) nach einem der Ansprüche 1 oder 2, **dadurch gekennzeichnet, dass** die Aufnahme zwischen der Zündvorrichtung und dem Reservoir (5) platziert ist, die den flüssigen Wirkstoff enthält.

4. Vorrichtung (1) nach einem der vorstehenden Ansprüche, **dadurch gekennzeichnet, dass** die Zündvorrichtung der pyrotechnischen Ladung (62) eine Schlagvorrichtung (3) des Zünders umfasst.

5. Vorrichtung (1) nach Anspruch 4, **dadurch gekennzeichnet, dass** die Aufnahme des Körpers (2), die in der Lage ist, die Patrone (6) aufzunehmen, zwischen der Schlagvorrichtung (3) und einer Gasexpansionskammer (4) platziert ist, die stromaufwärts vom Reservoir (5) liegt.

6. Vorrichtung (1) nach Anspruch 5, **dadurch gekennzeichnet, dass** der Körper (2) einen ersten hohlen Teil und einen zweiten hohlen Teil umfasst, die zwei parallelen Achsen (A1, A2) folgend angeordnet und über eine Leitung verbunden sind, wobei diese Leitung die Aufnahme der Patrone (6) und die Gasexpansionskammer (4) abgrenzt.

7. Vorrichtung (1) nach Anspruch 6, **dadurch gekennzeichnet, dass** die Patrone (6) so in der Aufnahme des Körpers (2) platziert wird, dass der Zünder (60) in der Achse der Schlagvorrichtung (3) liegt, und dass die Kappe (61) in der Achse der Gasexpansionskammer (4) liegt.

## Claims

1. A needleless injection device (1) comprising a body (2) supporting or delimiting a plurality of elements forming a circuit of elements, said circuit comprising an initiating device, a gas generating cartridge (6), a reservoir (5) containing an active ingredient to be injected and a system for injecting the active ingredient, the body (2) including a housing intended to receive said cartridge (6), said housing being accessible from the outside so as to be able to directly insert the cartridge (6) into the circuit of elements, independently of the other elements, the gas generating cartridge (6) being a pyrotechnic cartridge (6) comprising a pyrotechnic charge (62), said cartridge (6) including a primer (60),
the body (2) including an opening communicating with the housing,
the circuit of elements formed in an inverted U-shape including two parallel branches interconnected by a perpendicular transverse branch,
the introduction of the cartridge (6) in the circuit being carried out perpendicularly to the axis of symmetry of the U-shape formed by the circuit,
this device being **characterized in that** the cartridge (6) has an L-shaped conduit and **in that**, once inserted, its shape follows a right angle present between one of the parallel branches of the inverted U-shape formed by the circuit and its transverse branch, and **in that** said conduit is sealed at one of its ends by the primer (60) and at its other end by a frangible cap (61).

2. The device (1) according to claim 1, **characterized in that** the cartridge (6), once in place in the housing, tightly seals the opening (20) with respect to the outside.

3. The device (1) according to any of claims 1 or 2, **characterized in that** the housing is placed between the initiating device and the reservoir (5) containing the liquid active ingredient.

4. The device (1) according to any one of the preceding claims, **characterized in that** the device for initiating the pyrotechnic charge (62) includes a device (3) for striking the primer.

5. The device (1) according to claim 4, **characterized in that** the housing of the body (2), capable of receiving the cartridge (6), is placed between the striker device (3) and a gas expansion chamber (4) located upstream of the reservoir (5).

6. The device (1) according to claim 5, **characterized in that** the body (2) comprises a first hollow portion and a second hollow portion disposed along two parallel axes (A1, A2) and connected by a conduit, this conduit delimiting the housing of the cartridge (6) and the gas expansion chamber (4).

7. The device (1) according to claim 6, **characterized in that** the cartridge (6) is placed in the housing of the body (2) such that the primer (60) is located in the axis of the striker device (3) and that the cap (61) is located in the axis of the gas expansion chamber (4).
